# EUROPEAN PATENT SPECIFICATION

(11) **EP 3 773 767 B1**
(45) Date of publication and mention of the grant of the patent: **08.11.2023**
(21) Application number: 19717616.7
(22) Date of filing: 27.03.2019
(51) Int. Cl.: A61L 27/06, A61L 27/46, A61L 27/24, A61L 27/54, A61C 8/00, A61C 8/02

(54) **A SHAPED BLOCK COMPRISING COLLAGEN**
GEFORMTER BLOCK MIT KOLLAGEN
BLOC FAÇONNÉ COMPRENANT DU COLLAGÈNE

(30) Priority: 27.03.2018 IL 25839218
(43) Date of publication of application: 17.02.2021
(73) Proprietor: Datum Dental Ltd., Lod 7120101 (IL)
(72) Inventor: BAYER, Thomas, 6248310 Tel Aviv (IL)
(74) Representative: Pearl Cohen Zedek Latzer Baratz UK LLP
(86) International application number: PCT/IL2019/050354
(87) International publication number: WO 2019/186557

(56) References cited:
- WO-A2-2009/151614
- US-A1- 2012 316 646
- US-A1- 2014 377 323
- US-A1- 2016 008 509
- ATSUTA IKIRU ET AL: "Soft tissue sealing around dental implants based on histological interpretation", JOURNAL OF PROSTHODONTIC RESEARCH, ELSEVIER, AMSTERDAM, NL, vol. 60, no. 1, 23 December 2015 (2015-12-23), pages 3-11, XP029386098, ISSN: 1883-1958, DOI: 10.1016/J.JPOR.2015.07.001

## Description

### FIELD OF THE INVENTION

This invention is directed to a shaped block, a dental implant abutment comprising an implant, an abutment and a shaped block, wherein the shaped block comprises a dried collagen matrix; and a method of preparation thereof.

### BACKGROUND OF THE INVENTION

Gingival inflammation around dental implants (peri implant mucositis) often leads to an irreversible bone loss (peri implantitis) that if not properly treated, may lead to implant loss. It is estimated to affect about -30% of all dental implants and is of major concern to patients and doctors.

One of many potential etiological factors for the development of peri implantitis is bacterial colonization within the biologic width i.e. the minimal distance between the implant margin and the alveolar bone crest. It is often correlated to inadequate soft tissue thickness which was shown to increase bone resorption following implant abutment connection. Another widely studied factor is soft tissue seal around implant abutments which is often characterized by fibroblasts and epithelial cells' attachment to titanium or zirconia. This essential attachment may be lost when repeated connection and disconnection of abutments is practiced during implant restorative phase.

The need to augment and reinforce the soft tissue attachment and thickness around dental implants yielded several surgical techniques, mostly based on harvesting dense autogenous connective tissue and implanting it next to implants at risk or in the esthetic zone. Limited surgical skills limit these procedures only to highly skilled surgeons. Hence, there is a need for a solution that involves the use of a medical device that will be effective and easy to use. This device should be able to support soft tissue augmentation and allow formation and maintenance of the biologic width.

This invention provides a shaped block comprising a dried collagen matrix. This block can be shaped in various shapes, e.g. an O-ring and was found to form and augment a connective tissue seal around dental-implant abutments.

WO 2009/151614 discloses a resilient dental system and methods thereof for dental restoration.

US 2016/008509 details novel collagen materials and specifically collagen membranes, tubes and threads, and methods for obtaining same.

### SUMMARY OF THE INVENTION

The present invention provides a dental implant abutment comprising an implant, an abutment and a shaped block comprising dried cross-linked collagen matrix; wherein the shaped block is positioned between the abutment and the implant such that it is in direct contact at least partially with the implant and with the abutment; wherein the shaped block is shaped such that when implanted the shaped block is in partial and/or full contact with bone and/or soft tissue surrounding the abutment. In another embodiment, the positioning of the shaped block within the dental-implant abutment allows a base for soft/hard tissue ingrowth. In another embodiment, sliding motion of shaped block between implant and abutment allows bridging the abutment and implant with the shaped block, giving rise to supported tissue ingrowth.

The present invention further provides a dental implant abutment for use in the stimulation of bone or soft tissue growth, where the dental implant abutment comprises an implant, an abutment and a shaped block comprising dried cross-linking collagen matrix; wherein the shaped block is positioned between the abutment and the implant such that it is in direct contact at least partially with the implant and with the abutment; the shaped block of the dental implant is shaped such that when implanted it is in partial and/or full contact with the bone and/or soft tissue surrounding the dental implant abutment; and when implanted the dental implant abutment provides space and environment for cell ingrowth.

In an embodiment, the shaped block further comprises hydroxyapatite. In an embodiment, the shaped block further comprises titanium.

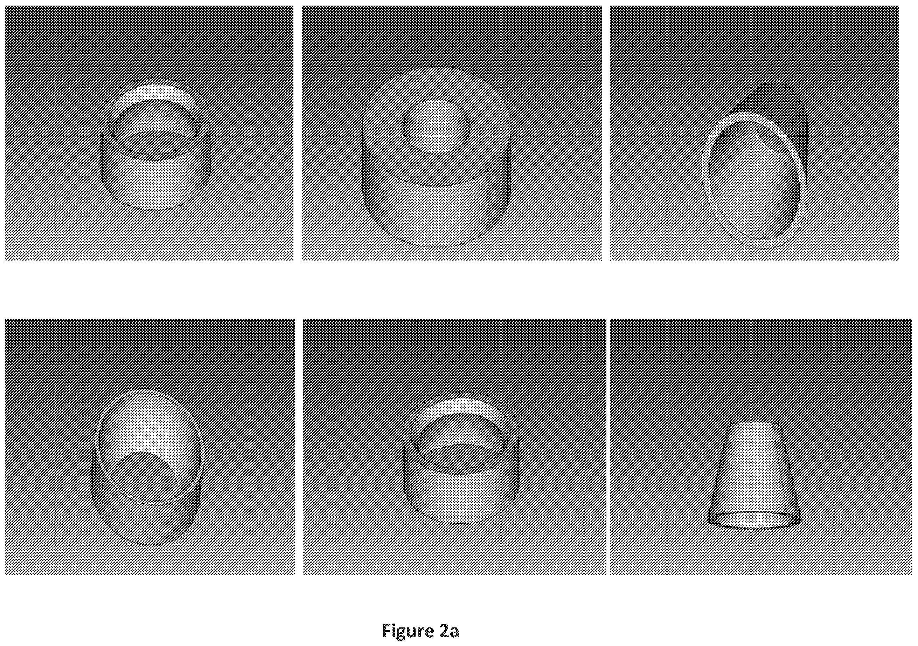

In another embodiment, the shaped block is shaped as an O-ring-like, a sleeve-like or a tube-like (Figure 2a).

In an embodiment, the shaped block further comprises a pharmaceutically active agent. In an embodiment, the agent comprises antibacterial agents, antifungal agents, anti-inflammatory agents, antibiotic agents, vitamins or any combination thereof.

The present invention also provides a method of preparing a shaped block comprising dried cross-linked collagen matrix, wherein the method comprises:
a. providing a dried cross-linked collagen matrix of this invention; and
b. carving out a shaped block from said dried matrix.

In an embodiment, the invention is direct to a method of preparing a dried crosslinked collagen matrix, wherein the method comprises:
(i) providing an acidic solution of collagen, followed by neutralization the solution;
(ii) concentrating the solution of step (i);
(iii) lyophilizing the concentrated mixture of step (ii), thereby obtaining a dried collagen composition;
(iv) incubating the composition with a crosslinker and a first solvent;
(v) washing the incubated composition of step (iv) with a second solvent; and
(vi) lyophilizing the washed composition of step (v);
thereby obtaining a dried cross-linked collagen matrix.

The present invention also provides a method of preparing a shaped block of the dental implant abutment as described hereinabove, wherein the method comprises:
(i) providing an acidic solution of collagen, followed by neutralization the solution;
(ii) concentrating the solution of step (i); and pouring it into a mold with a pre-designed block shape;
(iii) lyophilizing the concentrated mixture of step (ii), thereby obtaining a dried collagen composition, optionally comprising hydroxyapatite;
(iv) incubating the composition with a crosslinker, a first solvent and optionally with a pharmaceutically active agent;
(v) washing the incubated composition of step (iv) with a second solvent; and
(vi) lyophilizing the washed composition of step (v);
thereby obtaining a shaped block of the dental implant abutment as described hereinabove.

In one embodiment, the acidic solution in step (i) additionally comprises a crosslinker and the composition including the crosslinker added in step (i) is incubated in step (iv).

In one embodiment, a pharmaceutical agent is added to the incubated composition of step (iv) prior to the washing step (v).

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

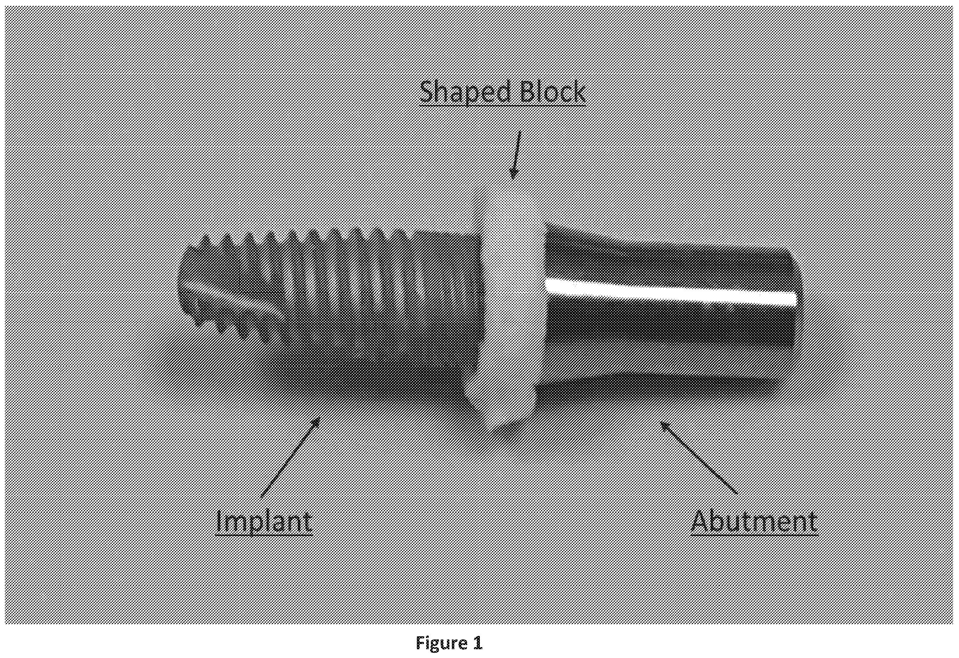

**Figure 1** depicts a dental implant abutment of the invention, comprising an implant, an abutment and a shaped block, denoted by arrows.
**Figures 2a-2b** depict shaped blocks of the invention. **Figure 2a**: drawing of such shapes; and **Figure 2b**: shaped blocks prepared according to embodiments of the invention.

It will be appreciated that for simplicity and clarity of illustration, elements shown in the Figures have not necessarily been drawn to scale. For example, the dimensions of some of the elements may be exaggerated relative to other elements for clarity. Further, where considered appropriate, reference numerals may be repeated among the Figures to indicate corresponding or analogous elements.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

### Shaped Block and uses thereof

The present invention provides a dental implant abutment comprising an implant, an abutment and a shaped block comprising dried cross-linked collagen matrix, wherein said shaped block is positioned between the abutment and the implant such that it is in direct contact at least partially with the implant and with the abutment; wherein the shaped block is shaped such that when implanted the shaped block is in partial and/or full contact with bone and/or soft tissue surrounding the abutment.

The present invention provides a dental implant abutment for use in the stimulation of bone or soft tissue growth, wherein said dental implant abutment comprises an implant, an abutment and a shaped block comprising dried cross-linked collagen matrix; said shaped block is positioned between the abutment and the implant such that it is in direct contact at least partially with the implant and with the abutment; the shaped block of the dental implant is shaped such that when implanted it is in partial and/or full contact with the bone and/or soft tissue surrounding the dental implant abutment; and when implanted the dental implant abutment provides space and environment for cell ingrowth.

In one aspect, the shaped block comprises dried collagen matrix. In one embodiment, the dried collagen matrix comprises a crosslinked collagen. In one embodiment, the shaped block comprises dried cross-linked collagen matrix. In another embodiment, the shaped block further comprises hydroxyapatite, titanium, pharmaceutically active agent or any combination thereof.

In another aspect, the shaped block is for use as an add-on element/unit for medical devices, implants, device attachments or any combination thereof, wherein the shaped block comprises a dried cross-linked collagen matrix.

In another embodiment, the shaped block is used as an add-on element/unit for medical devices. In another embodiment, the shaped block is used as an add-on element/unit for implants. In another embodiment, the shaped block is used as an add-on element/unit for device attachments. In another embodiment, any medical device as known in the art can be used. In another embodiment, implants are dental implant bodies. In another embodiment, any dental implant bodies as known in the art can be used. In another embodiment, examples for device attachments include prosthetics, shaped screws, shaped plates, shaped wires or any other attachment for bone/jar reconstruction.

In some embodiments, the term "add-on element/unit" refers to a shaped physical product that can be added to a known, other apparatus, device or product. In other embodiments, the addition is implemented by a person skilled in the art. In other embodiments, the addition comprises the following non-limiting actions: putting the add-on element/unit on or in proximity to the other device, manipulating them chemically to afford a robust connectivity in between them (e.g. welding, melting and solidification, dissolving and precipitation), manipulating them mechanically (e.g. screwing, rubbing) and any combination thereof. In another embodiment, the add-on unit/element is a shaped block which is added on a dental implant abutment, on wires, on plates, on screws or on clamps or on any other device, apparatus or product as known in the art.

In some embodiments, the term "block" refers to some physical extent of a solid matter. In other embodiments, the solid matter is dry and does not comprise any solvent or liquid. In other embodiments, the solid matter comprises at least one sole component. In another embodiment, examples of a component include: a chemical compound, small molecule, metal, alloy, composite material, biomaterial, polymer and organometallic complex. In another embodiment, the solid matter comprises more than one component as selected from the foregoing list. In another embodiment, the block comprises a biocompatible material or composition. In another embodiment, the block is a biopolymer or a protein. In other embodiments, the physical attributes of the block afford its design, molding, carving out or engineering as known in the art into a desired shape. In another embodiment, non -limiting examples of attributes of the block include the following characteristics: dense, porous or nonporous, viscous, rigid, soft or moldable. In another embodiment, the block has a porosity of between 10-90%. In another embodiment, the block has a porosity of between 10-20%. In another embodiment, the block has a porosity of between 20-30%. In another embodiment, the block has a porosity of between 30-40%. In another embodiment, the block has a porosity of between 40-50%. In another embodiment, the block has a porosity of between 50-60%. In another embodiment, the block has a porosity of between 60-70%. In another embodiment, the block has a porosity of between 70-80%. In another embodiment, the block has a porosity of between 80-90%. In another embodiment, the block has a porosity of between 10-30%. In another embodiment, the block has a porosity of between 30-50%. In another embodiment, the block has a porosity of between 50-70%. In another embodiment, the block has a porosity of between 70-90%.

In some embodiments, the term "shaped block‴ refers to a block which is provided in some shape or form, i.e. designed, engineered, manufactured or prepared as known in the art to provide some two or three-dimensional structure of such block. In other embodiments, examples of the two or three-dimensional structure include: square, circle, triangle, o-ring, sleeve, tube, pyramid, box, cuboid, cylinder, cone, prism and any other structure containing a hole within. In other embodiments, any possible design, engineering, manufacturing or preparation process can be applied in order to provide the shaped block.

In some embodiments, the term "collagen" refers to a biopolymer organized in a fibrillar networks or other non-fibrillar superstructures, and it is the main component of connective tissue within the human or numerous animals' body. Numerous types of collagen are known and found naturally, including types I-V. In some embodiments, collagen has a fibrillar (such as Type I) or non-fibrillar structure.

In one embodiment, the collagen used in the methods, uses and shape blocks of this invention refer to native collagen, fibrillar collagen, fibrillar atelopeptide collagen, lyophilized collagen, collagen obtained from animal sources, human collagen, recombinant collagen, pepsinized collagen, reconstituted collagen and any combination thereof. In another embodiment, the collagen includes fibrillar collagen reconstituted from monomolecular atelopeptide collagen. In another embodiment, the collagen is atelopeptide fibrillar collagen obtained by reconstituting monomolecular atelopeptide collagen obtained by proteolytic digestion of native collagen.

In some embodiments, the term "cross-linked collagen" refers to a covalent network comprising biopolymer chains of collagen connected covalently and inter-molecularly with crosslinkers.

In some embodiments, the term "crosslinkers" refers to small molecules or polymers comprising at least two ends that can covalently connect polymeric/oligomeric chains and thereby crosslink these chains.

In some embodiments the collagen is cross-linked by a sugar. In another embodiment, the sugar is selected from glycerose (glyceraldehyde), threose, erythrose, lyxose, xylose, arabinose, ribose, allose, altrose, glucose, mannose, gulose, idose, galactose, talose and any combination thereof.

In another embodiment, the sugar is a disaccharide.

In another embodiment, the disaccharide is selected maltose, lactose, sucrose, cellobiose, gentiobiose, melibiose, turanose, trehalose and any combination thereof.

In another embodiment, the positioning of the shaped block within the dental-implant abutment allows a base for soft/hard tissue ingrowth. In another embodiment, sliding motion of shaped block between implant and abutment allows bridging the abutment and implant with the shaped block, giving rise to supported tissue ingrowth. In another embodiment, the shaped block of the dental implant abutment is in partial and/or full contact with the bone and/or soft tissue surrounding the dental implant abutment.

In another embodiment, the implant is partially in contact with the shaped block. In another embodiment, the implant is covered completely by the shaped block. In one embodiment, any implant of the dental implant abutment of the invention can be utilized, provided or made, in any method as known in the art. In another embodiment, the implant is made from a material comprising polymers, ceramics, metals (e.g. Ti, Zr) or alloys or any combination thereof. In another embodiment, the shape/size of the implant comprises conical, cylindrical, plate, wire thread, hole(s)-containing shapes or any combination thereof. In one embodiment, any abutment of the dental implant abutment of the invention can be utilized, provided or made in any method as known in the art. In another embodiment, the abutment is made from a material comprising polymers, ceramics, metals (e.g. Ti, Zr) or alloys or any combination thereof. In another embodiment, the shape/size of the abutment comprises cylinders, cones, cubes or any combination thereof. In some embodiments, examples of polymers for abutments and/or implants include: polyurethane, polymethylmethacrylate, polysiloxanes, polylactic acid, polyacrylamides, any combination thereof and any other biocompatible polymer. In some embodiments, examples of ceramics for abutments and/or implants include: zirconia, alumina, titania, calcium phosphates, any combination thereof and any other biocompatible ceramic. In some embodiments, examples of metals or alloys for abutments and/or implants include: Ti, Zr, Ni, NiTi, any combination thereof and any other biocompatible metal or alloy.

In yet another aspect, the dental implant abutment of the invention is for use in the stimulation of bone or soft tissue growth. In one embodiment, the dental implant abutment of the invention provides space and environment for cell ingrowth. The soft but fitting nature of the material allows a closed fitting onto the surface of the abutment and the implant as well as onto the surrounding soft and bone tissue. This proximity in combination with the bone and soft tissue conductive properties allows a fast and effective incorporation of the abutment.

In another embodiment, the shaped block further comprises hydroxyapatite, titanium, pharmaceutically active agent or any combination thereof.

In other embodiments, examples for the pharmaceutically active agent include antibacterial agents, antifungal agents, anti-septic agents, anti-inflammatory agents, antibiotic agents, vitamins or any combination thereof. In another embodiment, any agent as known in the art within the foregoing list can be utilized.

In other embodiments, examples for the active agent include antibacterial agents, antifungal agents, anti-septic agents, anti-inflammatory agents, antibiotic agents, vitamins and vitamers and any combination thereof. In another embodiment, any agent as known in the art within the foregoing list can be utilized.

In another embodiment, examples of antibacterial agents include: Amikacin, Gentamicin, Kanamycin, Neomycin, Netilmicin, Tobramycin, Paromomycin, Arbekacin, Plazomicin, Streptomycin, Apramycin , Geldanamycin, Herbimycin, Loracarbef, Faropenem, Ertapenem, Doripenem, Imipenem, Meropenem, Cefazolin, Cefacetrile, Cefadroxil, Cephalexin, Cefaloglycin, Cefalonium, Cefaloridine, Cefalotin, Cefapirin, Cefatrizine, Cefazedone, Cefazaflur, Cefradine, Cefroxadine, Ceftezole, Cefaclor, Cefamandole, Cefminox, Cefonicid, Ceforanide, Cefotiam, Cefprozil, Cefbuperazone, Cefuroxime, Cefuzonam, Cephamycin, Cefoxitin, Cefotetan, Cefmetazole, Carbacephem, Cefixime, Ceftazidime, Ceftriaxone, Cefcapene, Cefdaloxime, Cefdinir, Cefditoren, Cefetamet, Cefmenoxime, Cefodizime, Cefoperazone, Cefotaxime, Cefpimizole, Cefpiramide, Cefpodoxime, Cefsulodin, Cefteram, Ceftibuten, Ceftiolene, Ceftizoxime, Oxacephem, Cefepime, Cefozopran, Cefpirome, Cefquinome, Ceftiofur, Cefquinome, Cefovecin, CXA-101, Ceftaroline, Ceftobiprole, Clindamycin, Lincomycin, Azithromycin, Clarithromycin, Dirithromycin, Erythromycin, Roxithromycin, Troleandomycin, Telithromycin, Spectinomycin, Solithromycin, Aztreonam , Furazolidone, Nitrofurantoin, Amoxicillin, Ampicillin, Azlocillin, Carbenicillin, Cloxacillin, Dicloxacillin, Flucloxacillin, Mezlocillin, Methicillin, Nafcillin, Oxacillin, Penicillin G, Penicillin V, Piperacillin, Temocillin, Ticarcillin, iprofloxacin, Enoxacin, Gatifloxacin, Levofloxacin, Lomefloxacin, Moxifloxacin, Nalidixic acid, Levonadifloxacin, Norfloxacin, Ofloxacin, Trovafloxacin, Grepafloxacin, Sparfloxacin, Temafloxacin, Delafloxacin, Mafenide, Sulfonamidochrysoidine, Sulfacetamide, Sulfadiazine, Sulfamethizole, Sulfamethoxazole, Sulfasalazine, Sulfisoxazole, Trimethoprim, emeclocycline, Doxycycline, Minocycline, Oxytetracycline, Tetracycline, Tigecycline, Tedizolid, Linezolid, Ranbezolid, Torezolid, Radezoli, any combination thereof and any pharmaceutically acceptable salt thereof.

In another embodiment, examples of antifungal agents include: terbinafine, naftifine, amphotericin B, butenafine, chloroxylenol, ciclopirox, flucytosine, caspofungin, griseofulvin, clotrimazole, fluconazole, itraconazole, ketoconazole, miconazole, oxiconazole, nystatin, undecylenic acid, any combination thereof and any pharmaceutically acceptable salt thereof.

In another embodiment, examples of anti-septic agents include: naftifine, tolnaftate, mediocidin, candicidin, trichomycin, hamycin, aurefungin, ascosin, ayfattin, azacolutin, trichomycin, levorin, heptamycin, candimycin, griseofulvin, pradimicins, benanomicin; ambisome; nikkomycin Z; flucytosine, perimycin, any combination thereof and any pharmaceutically acceptable salt thereof.

In another embodiment, examples of anti-inflammatory agents include: aspirin, ibuprofen, naproxen, celecoxib, diclofenac, ketoprofen, ketorolac, oxaprozin, salsalate, sulindac, any combination thereof and any pharmaceutically acceptable salt thereof.

In another embodiment, examples of antibiotics include penicillin, cephalosporin, ciprofloxacin, erythromycin, any combination thereof and any pharmaceutically acceptable salt thereof.

In another embodiment, examples of vitamins include: vitamin a, retinol, retinal, carotenoid, vitamin B1, thiamine, vitamin B2, riboflavin, vitamin B3, niacin, niacinamide, nicotinamide, riboside, vitamin B5, pantothenic acid, vitamin B6, pyridoxine, pyridoxamine, pyridoxal, vitamin B7, biotin, vitamin B9, folates, vitamin B12, cyanocobalamin, hydroxocobalamin, methylcobalamin, adenosylcobalamin, vitamin C, ascorbic acid, vitamin D, cholecalciferol (D3), ergocalciferol (D2), vitamin E, tocopherols, tocotrienols, vitamin K, phylloquinone, menaquinones, any combination thereof and any pharmaceutically acceptable salt thereof.

In some embodiments, the term "hydroxyapatite" refers to the naturally occurring calcium mineral with the formula of Ca₅(PO₄)₃(OH) or Ca₁₀(PO₄)₆(OH)₂. In other embodiments, hydroxyapatite can be prepared or obtained via any known method in the art. In another embodiment, bulk or nanoparticulate hydroxyapatite is utilized and/or prepared.

In some embodiments, the term "titanium" refers to a titanium metal or alloy. In other embodiments, titanium is alloyed, with, for example, Zr and/or Ni. In another embodiment the titanium is biocompatible. In another embodiment, the titanium is a biocompatible titanium alloy. In other embodiments, the titanium metal is of any shape or form as known in the art. In another embodiment, the titanium or alloyed titanium may have a shape memory function. In another embodiment, the titanium metal shape or form comprises bulk metal, surface thereof or nanoparticulate matter. In another embodiment, bulk metal is rod shaped, plate shaped, cube shaped or shaped in any other physical form as known in the art.

In some embodiments, the term "nanoparticulate matter" (e.g. hydroxyapatite or titanium) refers to a matter which has at least one physical nanometric dimension. In another embodiment, nanoparticulate matter is shaped as nanoparticles, nanospheres, nanocubes, nanoplates, nanoribbons, nanowires, nanorods or in any other nanometric shape as known in the art.

### Method of preparing a Shaped Block of this invention

In one further aspect, the invention also provides a method of preparing a shaped block comprising dried cross-linked collagen matrix of this invention wherein the method comprises:
a. providing a dried cross-linked collagen matrix of this invention; and
b. carving out a shaped block from said dried matrix.

In another embodiment, the dried cross-linked collagen matrix comprises hydroxyapatite. In another embodiment, the dried cross-linked collagen matrix comprises titanium. In another embodiment, the cross-linked collagen matrix comprises a pharmaceutically active agent. In another embodiment, the dried cross-linked collagen matrix comprises hydroxyapatite, titanium, a pharmaceutically active agent or any combination thereof.

In one embodiment, this invention is directed to a method of preparing a dried crosslinked collagen matrix wherein the matrix comprises a cross-linked collagen and the method comprises:
(i) providing an acidic solution of collagen, followed by neutralization of the solution;
(ii) concentrating the solution of step (i);
(iii)lyophilizing the concentrated mixture of step (ii), thereby obtaining a dried collagen composition;
(iv)incubating the composition with a crosslinker and a first solvent;
(v) washing the incubated composition of step (iv) with a second solvent; and
(vi)lyophilizing the washed composition of step (v);
thereby obtaining a dried cross-linked collagen matrix of this invention.

In one embodiment, this invention is directed to a method of preparing a dried cross linked collagen matrix, wherein the matrix comprises a cross linked collagen and optionally hydroxyapatite, titanium, a pharmaceutically active agent or any combination thereof and the method comprises:
(i) providing an acidic solution of collagen, followed by neutralization of the solution wherein the neutralization solution optionally comprises hydroxyapatite;
(ii) concentrating the solution of step (i) and optionally adding titanium;
(iii)lyophilizing the concentrated mixture of step (ii), thereby obtaining a dried collagen composition;
(iv)incubating the composition with a crosslinker, a first solvent and optionally adding a pharmaceutically active agent;
(v) washing the incubated composition of step (iv) with a second solvent; and
(vi)lyophilizing the washed composition of step (v);
thereby obtaining a dried cross linked collagen matrix of this invention.

In one embodiment, this invention is directed to a method of preparing a dried cross linked collagen matrix, wherein the matrix comprises a cross linked collagen and hydroxyapatite and the method comprises:
(i) providing an acidic solution of collagen, followed by neutralization of the solution, wherein the neutralization solution comprises hydroxyapatite;
(ii) concentrating the solution of step (i);
(iii)lyophilizing the concentrated mixture of step (ii), thereby obtaining a dried collagen composition;
(iv)incubating the composition with a crosslinker and a first solvent;
(v) washing the incubated composition of step (iv) with a second solvent; and
(vi)lyophilizing the washed composition of step (v);
thereby obtaining a dried cross-linked collagen matrix of this invention.

In one embodiment, this invention is directed to a method of preparing a dried cross linked collagen matrix, wherein the matrix comprises a cross linked collagen and a pharmaceutically active agent and the method comprises:
(i) providing an acidic solution of collagen, followed by neutralization of the solution;
(ii) concentrating the solution of step (i);
(iii)lyophilizing the concentrated mixture of step (ii), thereby obtaining a dried collagen composition;
(iv)incubating the composition with a crosslinker, a first solvent and a pharmaceutically active agent;
(v) washing the incubated composition of step (iv) with a second solvent; and
(vi)lyophilizing the washed composition of step (v);
thereby obtaining a dried cross-linked collagen matrix of this invention.

In one embodiment, this invention is directed to a method of preparing a dried cross linked collagen matrix, wherein the matrix comprises a cross linked collagen, titanium and a pharmaceutically active agent and the method comprises:
(i) providing an acidic solution of collagen, followed by neutralization of the solution;
(ii) concentrating the solution of step (i), adding titanium and
(iii)lyophilizing the concentrated mixture of step (ii), thereby obtaining a dried collagen composition;
(iv)incubating the composition with a crosslinker, a first solvent and a pharmaceutically active agent;
(v) washing the incubated composition of step (iv) with a second solvent; and
(vi)lyophilizing the washed composition of step (v);
thereby obtaining a dried cross-linked collagen matrix of this invention.

In another embodiment, compressing steps (applying mechanical pressure using a specialized equipment) are applied in addition to, or instead of the lyophilization steps.

In some embodiments, the carving out is done by any method as known in the art. In another embodiment, the carving out of the method of the invention is done by a CNC (Computer numerical control) machine, a laser cutting machine, a waterjet cutter, a driller or an abrasive device.

In some embodiments, the shaped block of the invention is provided in various shapes. In some embodiments, the blocks are shaped to allow an easy fitting of the block onto a dental abutment and to allow at least partial coverage of a dental implant once the abutment is placed into the implant. In another embodiment, the block is shaped as O-ring-like, a sleeve-like or a tube-like.

### Method of preparing a Shaped Block of this invention using a mold

The present invention further provides a method of preparing a shaped block comprising dried cross-linked collagen matrix, wherein the matrix comprises a cross linked collagen, optionally hydroxyapatite and optionally a pharmaceutically active agent and the method comprises:
(i) providing an acidic solution of collagen, followed by neutralization of the solution;
(ii) concentrating the solution of step (i); and pouring it into a mold with a pre designed block shape;
(iii)lyophilizing the concentrated mixture of step (ii), thereby obtaining a dried collagen composition, optionally comprising hydroxyapatite;
(iv)incubating the composition with a crosslinker, a first solvent and optionally with a pharmaceutically active agent;
(v) washing the incubated composition of step (iv) with a second solvent; and
(vi)lyophilizing the washed composition of step (v);
thereby obtaining a shaped block of this invention. In another embodiment, the neutralization solution of step (i) optionally comprises hydroxyapatite.

In one embodiment, the acidic solution in step (i) additionally comprises a crosslinker and the composition including the crosslinker added in step (i) is incubated in step (iv).

In one embodiment, a pharmaceutical agent is added to the incubated composition of step (iv) prior to the washing step (v).

In one embodiment, the method is directed to the preparation of a shaped block comprising dried cross-linked collagen matrix, wherein the matrix comprises a cross-linked collagen, optionally hydroxyapatite, optionally titanium and optionally a pharmaceutically active agent and the method comprises:
(i) providing an acidic solution of collagen, followed by neutralization of the solution wherein the neutralization solution optionally comprises hydroxyapatite;
(ii) concentrating the solution of step (i), optionally adding titanium; and pouring it into a mold with a pre designed block shape;
(iii)lyophilizing the concentrated mixture of step (ii), thereby obtaining a dried collagen composition, optionally comprising hydroxyapatite;
(iv)incubating the composition with a crosslinker, a first solvent and optionally with a pharmaceutically active agent;
(v) washing the incubated composition of step (iv) with a second solvent; and
(vi)lyophilizing the washed composition of step (v);
thereby obtaining a shaped block of this invention.

In another embodiment, compressing steps (applying mechanical pressure using a specialized equipment) are applied in addition to, or instead of the lyophilization steps.

In another embodiment, the mold is cooled to a freezing temperature of between -10 °C to -190 °C prior to the lyophilization step (iii). In another embodiment, the mold is cooled to a freezing temperature of between -10 to -80 °C for a period of between 0.5 to 24 hours followed by a lyophilization step (iii). In another embodiment, the cooling is carried out for between 0.5-24 hours. In another embodiment, the cooling is carried out for between 0.5-1 hours. In another embodiment, the cooling is carried out for between 1-2 hours. In another embodiment, the cooling is carried out for between 2-5 hours. In another embodiment, the cooling is carried out for between 5-10 hours. In another embodiment, the cooling is carried out for between 10-24 hours.

In another embodiment, the mold is shaped to afford the desired block shape, using a method comprising 3D printing, cast molding or any combination thereof.

### Method of preparing a Shaped Block of this invention via granulates

In one instance, the disclosure may be directed to a method of preparing a shaped block comprising dried cross-linked collagen matrix, wherein the matrix comprises a cross linked collagen, optionally hydroxyapatite, optionally titanium and optionally a pharmaceutically active agent and the method comprises:
(i) providing an acidic solution of collagen, followed by neutralization of the solution wherein the neutralization solution optionally comprises hydroxyapatite;
(ii) concentrating the solution of step (i) and optionally adding titanium;
(iii)incubating the composition with a crosslinker, a first solvent and optionally adding a pharmaceutically active agent;
(iv)washing the incubated composition of step (iv) with a second solvent;
(v) homogenizing, casting and milling the composition to obtain granulates of cross linked collagen;
(vi) wetting the granulates of step (v) by the first or second solvent and carving out to obtain a shaped block of this invention.

In some embodiments, the shaped block and/or dried cross linked collagen matrix prepared via the methods of this invention as described hereinabove - are used in the dental implant abutment of this invention, also described above; and/or they (block/matrix) are part of such dental implant abutment.

In some disclosed instances, the shaped block or blocks obtained by the methods of this invention can be milled to form granulates and then wetted by a first or second solvent and carved out to obtain a shaped block comprising cross-linked collagen matrix. In another instance, the size of the granulates are between 1 to 2000 microns.

In some embodiments, the collagen used in the methods of this invention within the solution of step "(i)" is selected from the following examples including: native collagen, fibrillar collagen, fibrillar atelopeptide collagen, lyophilized collagen, collagen obtained from animal sources, human collagen, recombinant collagen, pepsinized collagen, reconstituted collagen and any combination thereof. In another embodiment, the collagen includes fibrillar collagen reconstituted from monomolecular atelopeptide collagen. In another embodiment, the collagen is atelopeptide fibrillar collagen obtained by reconstituting monomolecular atelopeptide collagen obtained by proteolytic digestion of native collagen.

In another embodiment, the neutralization solution comprises a base or a buffer. In another embodiment, the neutralization solution further comprises hydroxyapatite. In another embodiment the buffer is selected from a phosphate buffered saline, NaHCO₃/Na₂CO₃ buffer, tris buffer or a tricine buffer or any other buffer that maintains a neutral pH. In another embodiment, the acidic solution comprises HCl, acetic acid, nitric acid, citric acid, sulfuric acid, phosphoric acid or any other acid as known in the art. In another embodiment, the basic solution comprises NaOH, KOH, NaHCOs, Na₂CO₃, Na₂HPO₄ or any other base as known in the art.

In some embodiments, the term "neutral pH" refers to a range of pHs which resembles the physiological pH in biological body and/or system; and it's defined between 6.5-7.5. In some other embodiments, a neutral pH is between 6.5-6.7. In some other embodiments, a neutral pH is between 6.7-6.9. In some other embodiments, a neutral pH is between 6.9-7.1. In some other embodiments, a neutral pH is between 7.1-7.3. In some other embodiments, a neutral pH is between 7.3-7.5. In some other embodiments, a neutral pH is between 7.1-7.2. In some other embodiments, a neutral pH is between 7.2-7.3. In some other embodiments, a neutral pH is between 7.3-7.4. In some other embodiments, a neutral pH is between 7.4-7.5.

In one embodiment, the concentrating step is done by centrifugation. In another embodiment, the centrifugation is carried out at a rate of between 50-20,000 RPM (rounds per minute). In another embodiment, centrifugation is carried out at a rate of between 50-100 RPM. In another embodiment, centrifugation is carried out at a rate of between 100-1,000 RPM. In another embodiment, centrifugation is carried out at a rate of between 1,000-5,000 RPM. In another embodiment, centrifugation is carried out at a rate of between 5,000-10,000 RPM. In another embodiment, centrifugation is carried out at a rate of between 10,000 -20,000 RPM. In another embodiment, centrifugation is carried out for between 1-120 minutes. In another embodiment, centrifugation is carried out for between 1-5 minutes. In another embodiment, centrifugation is carried out for between 5-10 minutes. In another embodiment, centrifugation is carried out for between 10-20 minutes. In another embodiment, centrifugation is carried out for between 20-50 minutes. In another embodiment, centrifugation is carried out for between 50-100 minutes. In another embodiment, centrifugation is carried out for between 100-120 minutes.

In another embodiment, the lyophilization of step (iii) is carried out for between 1-48 hours. In another embodiment, lyophilization of step (iii) is carried out for between 1-2 hours. In another embodiment, lyophilization of step (iii) is carried out for between 2-5 hours. In another embodiment, lyophilization of step (iii) is carried out for between 5-10 hours. In another embodiment, lyophilization of step (iii) is carried out for between 10-24 hours. In another embodiment, lyophilization of step (iii) is carried out for between 24-48 hours.

Following the lyophilization, a dried collagen composition of this invention is obtained. The dried composition is incubated with a crosslinker, a first solvent and optionally with a pharmaceutically active agent. The incubated composition is further washed with a second solvent and lyophilized to obtain the block collagen composition.

In another embodiment, the first and second solvents are the same or different and selected form any solvent as known in the art. In another embodiment, the solvent is selected from water, ethanol, saline, methanol, phosphate buffer saline or any combination thereof.

In another embodiment, the crosslinker can be any crosslinking agent as known in the art. In another embodiment, the crosslinker is a sugar. In another embodiment, the sugar is a compound represented by at least one of the following formulae I or II: 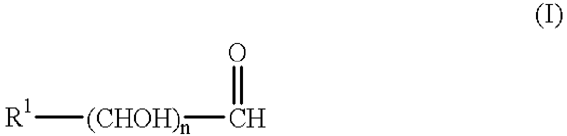 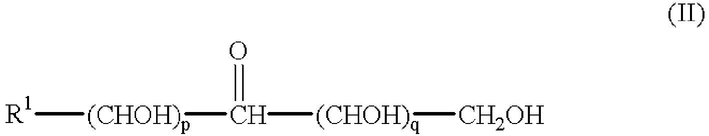 wherein:
R¹ is H or alkyl or alkenyl, an amino acid moiety, a peptide moiety, a saccharide moiety, a purine or a pyrimidine moiety, a phosphorylated purine or pyrimidine moiety;
n is an integer between 2-9, and
p and q are each independently an integer between 0-8, and the sum of p and q is at least 2 and not more than 8.

In another embodiment, the term "alkyl" group refers to a saturated aliphatic hydrocarbon, including straight-chain or branched-chain. In one embodiment, alkyl group is linear or branched. In another embodiment, alkyl is optionally substituted linear or branched. In one embodiment, the alkyl group has between 1-20 carbons. In one embodiment, the alkyl group has between 1-10 carbons. In one embodiment, the alkyl group has between 2-10 carbons. In one embodiment, the alkyl group has between 1-6 carbons. In one embodiment, the alkyl group has between 2-8 carbons. In another embodiment, examples of alkyl groups include methyl, ethyl, propyl, isopropyl, isobutyl, butyl, pentyl, 3-pentyl, hexyl heptyl, octyl and hexadecyl. In another embodiment, the alkyl group is optionally substituted by one or more halogens, hydroxides, alkoxides, carboxylic acids, phosphates, phosphonates, sulfates, sulfonates amidates, cyanates, and a nitro group.

In another embodiment, the term "alkenyl" group refers to an alkyl groups as described herein, having at least one carbon carbon double bond, including straight-chain and branched-chain groups. In one embodiment, the alkene has one double bond. In another embodiment, the alkene has more than one double bond. In another embodiment, the alkene has between 2-6 double bonds. In one embodiment, the alkene has 2-20 carbons. Examples include ethylenyl, propylenyl, 2-methylpropyl-1-enyl and butenyl.

In another embodiment, the term "amino acid" refers to an organic compound containing amine (-NH₂) and carboxyl (-COOH) functional groups, along with a side chain specific to each amino acid.. In another embodiment, any amino acid as known in the art can be utilized. In another embodiment, amino acid is alanine, arginine , asparagine, aspartic acid, cysteine, glutamine, glutamic acid, glycine, histidine, isoleucine, leucine, lysine, methionine, phenylalanine, proline, serine, threonine, tryptophan, tyrosine or valine.

In another embodiment, the term "peptide" refers to short chains of amino acids linked covalently via amide (peptide; -C(O)-N(H)-) bonds. In another embodiment, a peptide comprises between 2-20 amino acids. In another embodiment, a peptide is a dipepetide. In another embodiment, a peptide is a tripepetide. In another embodiment, a peptide is a tetrapepetide. In another embodiment, a peptide is a pentapepetide. In another embodiment, a peptide is a hexapepetide.

In another embodiment, the term "saccharide" refers to the group comprising sugars as described herein, cellulose and starch.

In another embodiment, the term "purine" refers to a heterocyclic aromatic organic compound that consists of a pyrimidine ring fused to an imidazole ring. Non limiting examples of purines include: purine, adenine, guanine, hypoxanthine, xanthine, theobromine, caffeine, uric acid and isoguanine.

In another embodiment, the term "pyrimidine" refers to a heterocyclic aromatic organic compound similar to pyridine but has an additional nitrogen within the aromatic ring so nitrogens are found in position 1, 3 of the ring. Examples of purines include: cytosine, thymine and uracil.

In another embodiment, the term "a phosphorylated purine or pyrimidine" refers to a purine or pyrimidine as described herein, wherein the purine or pyrimidine is connected to a phosphoryl group (the chemical entity PO₃^{x-}; "x" denoting any possible protonation state).

In another embodiment, the sugar is a naturally occurring reducing sugar.

In another embodiment, the sugar is a diose, a triose, a tetrose, a pentose, a hexose, a septose, an octose, a nanose, or a decose.

In another embodiment, the sugar is selected from glycerose (glyceraldehyde), threose, erythrose, lyxose, xylose, arabinose, ribose, allose, altrose, glucose, mannose, gulose, idose, galactose, talose and any combination thereof.

In another embodiment, the sugar is a disaccharide.

In another embodiment, the disaccharide is selected from maltose, lactose, sucrose, cellobiose, gentiobiose, melibiose, turanose, trehalose and any combination thereof.

Following the crosslinking and the washing steps with a first and second solution the composition is lyophilized. In another embodiment, the lyophilization of step (vi) is carried out for between 24 to 72 hrs and the block composition is obtained. In one embodiment, the block collagen composition is prepared using a mold with a pre-designed shape, thereby, a block shape collagen composition is obtained. In another embodiment, a mold is not used and a block collagen composition is obtained which is further carved out to a desired block shape collagen composition.

In some other embodiments, the designed mold and the carving out is planned, designed and/or engineered via computer-aided design (CAD) and/or computer-aided manufacturing (CAM) methods and software as known in the art of the invention.

In another embodiment, the carving out of the method of the invention is done by any method as known in the art. In another embodiment, the carving out of the method of the invention is done by a CNC (Computer numerical control) machine, a laser cutting machine, a waterjet cutter, a driller or an abrasive device.

In some embodiments, the shaped block of the invention is provided in various shapes. In some embodiments, the blocks are shaped to allow an easy fitting of the block onto a dental abutment and to allow at least partial coverage of a dental implant once the abutment is placed into the implant. In another embodiment, the block is shaped as O-ring-like, a sleeve-like or a tube-like (Figure 2a).

The following examples are presented in order to more fully illustrate the preferred embodiments of the invention. They should in no way, however, be construed as limiting the broad scope of the invention.

### EXAMPLES

### EXAMPLE 1

### Crosslinking of Collagen

A solution of molecular purified pepsinized Porcine Type I collagen (1-10 milligram/milliliter), prepared from Porcine tendons (commercially available from Pel-Freez, AR, U.S.A), was dissolved in 0.01M HCl and maintained at 4° C. It was neutralized by 0.1M NaOH to pH 7.2-7.4, poured into an appropriate mold, and incubated for 24 hours at a temperature ranging between 20-38° C. The matrix which is produced is then compressed by a piston to remove excess solution. The resulting collagen membranes were incubated for 11 days in PBS.

The fibrillated collagen was concentrated by centrifugation at 3000 rpm. All centrifugations (unless specifically stated otherwise) were done using a model RC5C centrifuge with a SORVALL SS-34 rotor commercially available from SORVALL^{®} Instruments DUPONT, USA. The fibrillated collagen concentration after centrifugation was brought to approximately 35 mg/mL by the use of 10 millimolar phosphate buffer solution (PBS pH 7.36). The mixture was poured into a stainless steel tray. The tray was transferred into the lyophilizer (Freeze dryer model FD 8 commercially available from Heto Lab Equipment DK-3450 Allerød, Denmark), pre-frozen for eight hours and lyophilized for 24 hours. The condenser temperature was -80°C. The shelf temperature during pre-freezing was -40°C. The shelf temperature during lyophilization was +35°C and the vacuum during lyophilization was approximately 0.01 bar.

200 mL of a solution containing 120 mL absolute ethanol (commercially available from Merck, Germany), 80 mL PBS buffer solution (10 mM, pH 7.36) and 3 gram of D(-) ribose (commercially available as Catalogue No. R7500 from Sigma, USA) were added to the dried (lyophilized) fibrillated collagen and incubated at 37 °C for 11 days to perform the ribose cross-linking of the collagen structure. The ribose cross-linked collagen products were washed exhaustively with DI water and lyophilized, using the same conditions as described above.

### EXAMPLE 2

### Crosslinking of Collagen-Hydroxyapatite

A solution containing purified collagen consists of atelocollagen monomers [pepsinized type I collagen (~3 mg/mL)], dissolved in 0.05 M acetic acid and maintained at 4° C was mixed with a slurry of 0.1 M NaOH containing hydroxyapatite (collagen/HA ratio: 95:5 - 70:30) and brought to a neutral pH. Then the solution was incubated under constant stirring for 24 hours at a temperature ranging between 20-37° C. The fibrillated collagen/HA mixture was concentrated by centrifugation at 3000 rpm for 15 min. The fibrillated collagen concentration after centrifugation was brought to approximately 35 mg/mL by the use of 10 millimolar phosphate buffer solution (PBS pH 7.36). The mixture was homogenized for 10 min at 100m rpm with Planetary Centrifugal Mixer ("THINKY MIXER" ARE-500; THINKY CORPORATION, Japan) poured into a stainless steel tray. The tray was transferred into the lyophilizer (Freeze dryer model FD 8 commercially available from Heto Lab Equipment DK-3450 Allerød, Denmark), pre-frozen for eight hours and lyophilized for 24 hours. The condenser temperature was -80°C. The shelf temperature during pre-freezing was -40°C. The shelf temperature during lyophilization was +35°C and the vacuum during lyophilization was approximately 0.01 bar.

200 mL of a solution containing 120 mL absolute ethanol (commercially available from Merck, Germany), 80 mL PBS buffer solution (10 mM, pH 7.36) and 3 gram of DL-glyceraldehyde (commercially available from Biosynth, Switzerland) were added to the dried (lyophilized) fibrillated collagen and incubated at 37 °C for 11 days to perform the ribose crosslinking of the collagen structure. The ribose cross-linked collagen products were washed exhaustively with DI water and lyophilized, using the same conditions as described above.

### EXAMPLE 3

### Carving out Collagen blocks

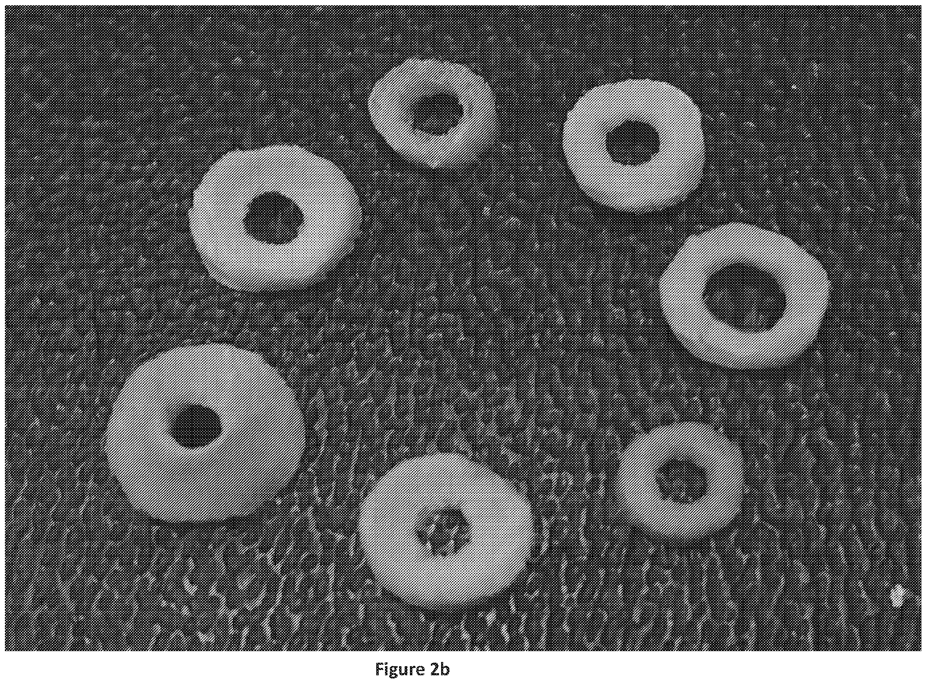

Blocks of the dried collagen matrix have been used to carve out desired shapes. These shapes were of O-ring-like appearance or sleeve-like or tube-like (Figure 2b). These structures easily fit onto an abutment and partially or fully covering the implant once the abutment is placed onto the implant.

The soft but fitting nature of the material allows a closed fitting onto the surface of the abutment and the implant as well as onto the surrounding soft and bone tissue.
This proximity in combination with the bone and soft tissue conductive properties will allow a fast and effective incorporation of the abutment.

## Claims

1. A dental implant abutment comprising an implant, an abutment and a shaped block comprising dried cross-linked collagen matrix, wherein said shaped block is positioned between the abutment and the implant such that it is in direct contact at least partially with the implant and with the abutment; wherein the shaped block is shaped such that when implanted it is in partial and/or full contact with bone and/or soft tissue surrounding the abutment.

2. A dental implant abutment for use in the stimulation of bone or soft tissue growth, wherein said dental implant abutment comprises an implant, an abutment and a shaped block comprising dried cross-linked collagen matrix; said shaped block is positioned between the abutment and the implant such that it is in direct contact at least partially with the implant and with the abutment; the shaped block of the dental implant is shaped such that when implanted it is in partial and/or full contact with the bone and/or soft tissue surrounding the dental implant abutment; and when implanted the dental implant abutment provides space and environment for cell ingrowth.

3. The dental implant abutment of claim 1 or the dental implant abutment for use of claim 2, wherein said shaped block further comprises hydroxyapatite.

4. The dental implant abutment of claim 1 or the dental implant abutment for use of claim 2, wherein said shaped block further comprises titanium.

5. The dental implant abutment according to any one of claims 1,3 or 4 or the dental implant abutment for use of claims 2-4, wherein said block has a shape of an O-ring-like, a sleeve-like or a tube-like.

6. The dental implant abutment according to any one of claims 1 or 3-5 or the dental implant abutment for use of claims 2-5, wherein the shaped block further comprises a pharmaceutically active agent.

7. The dental implant abutment of claim 6, or the dental implant abutment for use of claim 6, wherein said agent comprises antibacterial agents, antifungal agents, anti-inflammatory agents, antibiotic agents, vitamins or any combination thereof.

8. A method of preparing a shaped block of the dental implant abutment according to any one of claims 1-7, wherein the method comprises:
a. providing the dried cross-linked collagen matrix; and
b. carving out a shaped block from said dried matrix.

9. The method of claim 8, wherein the dried cross-linked collagen matrix is prepared by a method comprising:
(i) providing an acidic solution of collagen, followed by neutralization the solution;
(ii) concentrating the solution of step (i);
(iii) lyophilizing the concentrated mixture of step (ii), thereby obtaining a dried collagen composition;
(iv) incubating the composition with a crosslinker and a first solvent;
(v) washing the incubated composition of step (iv) with a second solvent; and
(vi) lyophilizing the washed composition of step (v);
thereby obtaining a dried cross-linked collagen matrix.

10. A method of preparing a shaped block of the dental implant abutment according to any one of claims 1-7, wherein the method comprises:
(i) providing an acidic solution of collagen, followed by neutralization the solution;
(ii) concentrating the solution of step (i); and pouring it into a mold with a pre-designed block shape;
(iii) lyophilizing the concentrated mixture of step (ii), thereby obtaining a dried collagen composition, optionally comprising hydroxyapatite;
(iv) incubating the composition with a crosslinker, a first solvent and optionally with a pharmaceutically active agent;
(v) washing the incubated composition of step (iv) with a second solvent; and
(vi) lyophilizing the washed composition of step (v);
thereby obtaining a shaped block of the dental implant abutment according to any one of claims 1-7.

11. The method according to claim 10, wherein in step (i) the acidic solution additionally comprises a crosslinker and wherein in step (iv) the composition including the crosslinker added in step (i) is incubated.

12. The method according to claim 10, wherein prior to the washing step (v) a pharmaceutical agent is added to the incubated composition of step (iv).

13. The method according to any one of claims 8-12, wherein said obtained shaped block has a shape of an O-ring-like, a sleeve-like or a tube-like.

14. The method according to any one of claims 8-9, wherein said carving out is done by a CNC machine, a laser cutting machine, a waterjet cutter, a driller, an abrasive device OR by using casting molds or 3D printer during the manufacturing process.

15. The method according to claim 10-12 wherein said mold is shaped using a method comprising 3D printing, cast molding or any combination thereof.

## Patentansprüche

1. Zahnimplantat-Abutment, umfassend ein Implantat, ein Abutment und ein Formteil, das eine getrocknete vernetzte Kollagenmatrix umfasst, wobei das Formteil zwischen dem Abutment und dem Implantat positioniert ist, derart, dass es mindestens teilweise mit dem Implantat und dem Abutment in einem direkten Kontakt ist; wobei das Formteil derart geformt ist, dass es, wenn es implantiert ist, in einem teilweisen und/oder vollen Kontakt mit dem Knochen und/oder dem Weichteilgewebe, das das Abutment umgibt, ist.

2. Zahnimplantat-Abutment zur Verwendung bei der Stimulation des Wachstums von Knochen- oder Weichteilgewebe, wobei das Zahnimplantat-Abutment ein Implantat, ein Abutment und ein Formteil, das eine getrocknete vernetzte Kollagenmatrix umfasst, umfasst, wobei das Formteil zwischen dem Abutment und dem Implantat positioniert ist, derart, dass es mindestens teilweise mit dem Implantat und dem Abutment in einem direkten Kontakt ist; wobei das Formteil des Zahnimplantats derart geformt ist, dass es, wenn es implantiert ist, in einem teilweisen und/oder vollen Kontakt mit dem Knochen und/oder dem Weichteilgewebe, das das Abutment umgibt, ist; und wobei das Zahnimplantat-Abutment, wenn es implantiert ist, Platz und eine Umgebung für das Einwachsen von Zellen bietet.

3. Zahnimplantat-Abutment nach Anspruch 1 oder Zahnimplantat-Abutment zur Verwendung nach Anspruch 2, wobei das Formteil ferner Hydroxyapatit umfasst.

4. Zahnimplantat-Abutment nach Anspruch 1 oder Zahnimplantat-Abutment zur Verwendung nach Anspruch 2, wobei das Formteil ferner Titan umfasst.

5. Zahnimplantat-Abutment nach einem der Ansprüche 1, 3 oder 4 oder Zahnimplantat-Abutment zur Verwendung nach Anspruch 2-4, wobei das Formteil eine Form ähnlich einem O-Ring, ähnlich einer Hülse oder ähnlich einer Röhre hat.

6. Zahnimplantat-Abutment nach einem der Ansprüche 1 oder 3-5 oder Zahnimplantat-Abutment zur Verwendung nach Anspruch 2-5, wobei das Formteil ferner einen pharmazeutisch aktiven Wirkstoff umfasst.

7. Zahnimplantat-Abutment nach Anspruch 6 oder Zahnimplantat-Abutment zur Verwendung nach Anspruch 6, wobei der Wirkstoff antibakterielle, antifungielle, antiinflammatorische und antibiotische Wirkstoffe, Vitamine oder eine Kombination davon umfasst.

8. Verfahren zum Herstellen eines Formteils des Zahnimplantat-Abutments nach einem der Ansprüche 1-7, wobei das Verfahren Folgendes umfasst:
a. Bereitstellen der getrockneten vernetzten Kollagenmatrix; und
b. Herausschälen des Formteils aus der getrockneten Matrix.

9. Verfahren nach Anspruch 8, wobei die getrocknete vernetzte Kollagenmatrix durch ein Verfahren, das Folgendes umfasst, hergestellt wird:
(i) Bereitstellen einer sauren Kollagenlösung, gefolgt von einer Neutralisation der Lösung;
(ii) Aufkonzentrieren der Lösung, die in Schritt (i) erhalten wurde;
(iii) Lyophilisieren des konzentrierten Gemisches, das in Schritt (ii) erhalten wurde, um dadurch eine getrocknete Kollagenzusammensetzung zu erhalten;
(iv) Inkubieren der Zusammensetzung mit einem Vernetzer und einem ersten Lösungsmittel;
(v) Waschen der inkubierten Zusammensetzung, die in Schritt (iv) erhalten wurde, mit einem zweiten Lösungsmittel; und
(vi) Lyophilisieren der gewaschenen Zusammensetzung, die in Schritt (v) erhalten wurde, um dadurch eine getrocknete vernetzte Kollagenmatrix zu erhalten.

10. Verfahren zum Herstellen eines Formteils des Zahnimplantat-Abutments nach einem der Ansprüche 1-7, wobei das Verfahren Folgendes umfasst:
(i) Bereitstellen einer sauren Kollagenlösung, gefolgt von einer Neutralisation der Lösung;
(ii) Aufkonzentrieren der Lösung, in Schritt (i) erhalten wurde; und Gießen derselben in eine Form, die eine vorab entworfene Formteil-Form hat;
(iii) Lyophilisieren des konzentrierten Gemisches, das in Schritt (ii) erhalten wurde, um dadurch eine getrocknete Kollagenzusammensetzung, wahlweise umfassend Hydroxyapatit, zu erhalten;
(iv) Inkubieren der Zusammensetzung mit einem Quervernetzer, einem ersten Lösungsmittel und wahlweise einem pharmazeutisch aktiven Wirkstoff;
(v) Waschen der inkubierten Zusammensetzung, die in Schritt (iv) erhalten wurde, mit einem zweiten Lösungsmittel; und
(vi) Lyophilisieren der gewaschenen Zusammensetzung, die in Schritt (v) erhalten wurde, um dadurch ein Formteil des Zahnimplantat-Abutments nach einem der Ansprüche 1-7 zu erhalten.

11. Verfahren nach Anspruch 10, wobei die saure Lösung aus Schritt (i) zusätzlich einen Quervernetzer umfasst, und wobei die Zusammensetzung aus Schritt (iv) einschließlich des Quervernetzers, der in Schritt (i) zugesetzt wurde, inkubiert wird.

12. Verfahren nach Anspruch 10, wobei der inkubierten Zusammensetzung aus Schritt (iv) vor dem Wasch-Schritt (v) ein pharmazeutischer Wirkstoff zugesetzt wird.

13. Verfahren nach einem der Ansprüche 8-12, wobei das erhaltene Formteil eine Form hat, die ähnlich einem O-Ring, ähnlich einer Hülse oder ähnlich einer Röhre ist.

14. Verfahren nach einem der Ansprüche 8-9, wobei das Herausschälen mittels eines CNC-Geräts, Laser-Schneidegeräts, Wasserstrahl-Schneidegeräts, eines Bohrers, eines Schleifgeräts oder durch Verwenden von Gussformen oder eines 3D-Druckers während des Fertigungsprozesses durchgeführt wird.

15. Verfahren nach einem der Ansprüche 10-12, wobei die Form durch Einsetzen eines Verfahrens, das 3D-Drucken, Formgießen oder eine Kombination davon umfasst, gebildet wird.

## Revendications

1. Pilier d'implant dentaire comprenant un implant, un pilier et un bloc façonné comprenant une matrice de collagène réticulé séchée, **caractérisé en ce que** ledit bloc façonné est positionné entre le pilier et l'implant de telle sorte qu'il soit en contact direct, au moins partiellement, avec l'implant et le pilier ; le bloc façonné étant façonné de telle sorte qu'une fois implanté, il soit en contact partiel et/ou total avec l'os et/ou les tissus mous entourant le pilier.

2. Pilier d'implant dentaire pour une utilisation dans la stimulation de la croissance des os ou des tissus mous, **caractérisé en ce que** ledit pilier d'implant dentaire comprend un implant, un pilier et un bloc façonné comprenant une matrice de collagène réticulé séchée ; ledit bloc façonné est positionné entre le pilier et l'implant de telle sorte qu'il soit en contact direct, au moins partiellement, avec l'implant et le pilier ; le bloc façonné de l'implant dentaire est façonné de telle sorte qu'une fois implanté, il soit en contact partiel et/ou total avec l'os et/ou les tissus mous entourant le pilier de l'implant dentaire ; et une fois implanté, le pilier de l'implant dentaire fournit un espace et un environnement propices à la croissance cellulaire.

3. Pilier d'implant dentaire selon la revendication 1, ou pilier d'implant dentaire pour une utilisation selon la revendication 2, **caractérisé en ce que** ledit bloc façonné comprend en outre de l'hydroxyapatite.

4. Pilier d'implant dentaire selon la revendication 1, ou pilier d'implant dentaire pour une utilisation selon la revendication 2, **caractérisé en ce que** ledit bloc façonné comprend en outre du titane.

5. Pilier d'implant dentaire selon l'une quelconque des revendications 1, 3 ou 4, ou pilier d'implant dentaire pour une utilisation selon les revendications 2 à 4, **caractérisé en ce que** ledit bloc a la forme d'un joint torique, d'un manchon ou d'un tube.

6. Pilier d'implant dentaire selon l'une quelconque des revendications 1 ou 3 à 5, ou pilier d'implant dentaire pour une utilisation selon les revendications 2 à 5, **caractérisé en ce que** le bloc façonné comprend en outre un agent pharmaceutiquement actif.

7. Pilier d'implant dentaire selon la revendication 6, ou pilier d'implant dentaire pour une utilisation selon la revendication 6, **caractérisé en ce que** ledit agent comprend des agents antibactériens, des agents antifongiques, des agents anti-inflammatoires, des agents antibiotiques, des vitamines ou toute combinaison de ceux-ci.

8. Procédé de préparation d'un bloc façonné du pilier d'implant dentaire selon l'une quelconque des revendications 1 à 7, **caractérisé en ce que** le procédé consiste à :
a. fournir la matrice de collagène réticulé séchée ; et
b. découper un bloc façonné à partir de ladite matrice séchée.

9. Procédé selon la revendication 8, **caractérisé en ce que** la matrice de collagène réticulé séchée est préparée selon un procédé consistant à :
(i) fournir une solution acide de collagène, puis neutraliser la solution ;
(ii) concentrer la solution de l'étape (i) ;
(iii) lyophiliser le mélange concentré de l'étape (ii), obtenant ainsi une composition de collagène séchée ;
(iv) incuber la composition avec un agent de réticulation et un premier solvant ;
(v) laver la composition incubée de l'étape (iv) avec un deuxième solvant ; et
(vi) lyophiliser la composition lavée de l'étape (v) ; obtenant ainsi une matrice de collagène réticulé séchée.

10. Procédé de préparation d'un bloc façonné du pilier d'implant dentaire selon l'une quelconque des revendications 1 à 7, **caractérisé en ce que** le procédé consiste à :
(i) fournir une solution acide de collagène, puis neutraliser la solution ;
(ii) concentrer la solution de l'étape (i) ; et la verser dans un moule ayant une forme de bloc prédéfinie ;
(iii) lyophiliser le mélange concentré de l'étape (ii), obtenant ainsi une composition de collagène séchée, comprenant éventuellement de l'hydroxyapatite ;
(iv) incuber la composition avec un agent de réticulation, un premier solvant et éventuellement avec un agent pharmaceutiquement actif ;
(v) laver la composition incubée de l'étape (iv) avec un deuxième solvant ; et
(vi) lyophiliser la composition lavée de l'étape (v) ; obtenant ainsi un bloc façonné du pilier d'implant dentaire selon l'une quelconque des revendications 1 à 7.

11. Procédé selon la revendication 10, **caractérisé en ce que**, à l'étape (i), la solution acide comprend en outre un agent de réticulation et **caractérisé en ce que**, à l'étape (iv), la composition comprenant l'agent de réticulation ajouté à l'étape (i) est incubée.

12. Procédé selon la revendication 10, **caractérisé en ce que**, avant l'étape de lavage (v), un agent pharmaceutique est ajouté à la composition incubée de l'étape (iv).

13. Procédé selon l'une quelconque des revendications 8 à 12, **caractérisé en ce que** ledit bloc façonné obtenu a la forme d'un joint torique, d'un manchon ou d'un tube.

14. Procédé selon l'une quelconque des revendications 8 à 9, **caractérisé en ce que** ladite découpe est réalisée par une machine CNC, une machine de découpe laser, une machine de découpe par jet d'eau, une perceuse, un dispositif abrasif OU en utilisant des moules de coulée ou une imprimante 3D pendant le processus de fabrication.

15. Procédé selon les revendications 10 à 12, **caractérisé en ce que** ledit moule est façonné en utilisant un procédé comprenant l'impression 3D, le moulage par coulée ou toute combinaison de ceux-ci.
